# EUROPEAN PATENT APPLICATION

(11) **EP 2 275 426 A1**
(43) Date of publication of application: **19.01.2011**
(21) Application number: 09738266.7
(22) Date of filing: 29.04.2009
(51) Int. Cl.: C07F 3/06, C07F 19/00, A61K 47/24, A61K 47/34, A61K 9/51

(54) **METALLO-ORGANIC SYSTEM FOR THE ENCAPSULATION AND RELEASE OF COMPOUNDS OF INTEREST, METHOD FOR OBTAINING SAME AND USES THEREOF**

(30) Priority: 29.04.2008 ES 200801230
(71) Applicant: Consejo Superior De Investigaciones Científicas, 28006 Madrid (ES); Institut Català De Nanotecnologia, 08193 Bellaterra (ES)
(72) Inventor: RUIZ MOLINA, Daniel, E-08193 Bellaterra (ES); MASPOCH COMAMALA, Daniel, E-08193 Bellaterra (ES); IMAZ GABILONDO, Inhar, E-08193 Bellaterra (ES)
(74) Representative: Pons Ariño, Angel
(86) International application number: PCT/ES2009/070128
(87) International publication number: WO 2009/133229

(57) **Abstract**

The present invention describes a metallo-organic system that includes: a metal ion salt or complex; at least one organic ligand; and a substance of interest to be encapsulated, belonging to the group comprising a biological entity, a drug, a vaccine, a diagnostic contrast agent, a marker, an organic compound, an inorganic compound, a metallo-organic compound or a nanomaterial or nanodevices. Also disclose is the process for obtaining same and the uses thereof for the release and/or protection and/or storage of said substances in the pharmaceutical, chemical, environmental, medical and industrial sectors.

## Description

### FIELD OF THE INVENTION

The present invention relates to the scientific and technical fields of Nanotechnology, Biotechnology, Medicine Materials Science and Chemistry in the sectors of manufacturing, encapsulation and functionalisation.

### STATE OF THE ART

The discovery of new systems that allow other substances to be encapsulated is one of the latest scientific challenges, due to the enormous variety of applications in areas as diverse as the pharmaceutical, medical or environmental sectors. Currently, there are basically six classes of systems that serve to encapsulate other substances. Said systems include dendrimers, micro and nanoparticles of organic polymers, liposomes, phospholipid micelles, cyclodextrins and carbon nanoparticles.

Until now, said systems have been used to encapsulate a large number and wide variety of substances. For example, the encapsulation of magnetic particles in organic polymer microparticles has created new systems for magnetic recording. Similarly, systems that encapsulate molecules that undergo changes with certain stimuli have been used as sensors.

One of the sectors in which micro- and nano-encapsulation have experienced the greatest surge, is the medical and pharmaceutical sector. In effect, the encapsulation of medicines, drugs, genes or any other biological element and their subsequent controlled release in specific places of the body are having enormous impacts on fields as diverse as cardiology, oncology, endocrinology, immunology, etc. These systems present a whole range of advantages in comparison with conventional drug administration systems because 1) they limit secondary effects due to the drug's selective release in the specific zones to be treated; 2) they reduce the amount of drug required; 3) they maintain prolonged levels of the drug; 4) they facilitate administration of the drug; and 5) they increase solubility of the drug. All these advantages are encouraging the already successful use of these systems in the treatment of diseases such as cancer and lung problems. Due to their importance, such systems are having a major repercussion on the world economy. For example, sale of these systems in the United States generated turnover of $54.2 billion in 2004, $64.1 billion in 2005 and $67.7 billion in 2006. This increase in earnings as well as the possibility of curing diseases that were fatal until now has initiated a very promising race towards the development and improvement of the most well-known drug encapsulation and release systems (liposomes, dendrimers and polymeric microparticles) and towards the discovery of new systems.

At the same time, in recent years, metallo-organic materials and more specifically coordination polymer macrocrystals have shown a great variety of properties (e.g. porosity, magnetism, electronic, fluorescence, etc.) and applications (e.g. ion exchange, absorption, sensors, etc.). Due to the enormous variety of geometries and properties displayed by these materials and the almost unlimited number of organic ligands and/or metal ions used, metallo-organic materials can be designed and prepared in the required compositions, dimensions and morphologies. For this reason, the recent emergence of new trends aimed at miniaturising these materials to micro- and nano-metric scales is unsurprising.

The synthesis of metallo-organic spherical particles made through infinite coordination polymerisation of metal ions bound by organic ligands is disclosed in international application W02007053181. These particles were exclusively synthesised with metal ions (Zn, Cu, Mn, Pb, Ni, Co, Cd, and Cr) and Schiff-base ligands, the synthesis consisting firstly of mixing a metal salt with formula M(O₂CCH₃)_{y} (M = Zn, Cu, Mn, Pb, Ni, Co, Cd and Cr) and a pre-synthesised metal complex and finally precipitating the metallo-organic particles using a non-polar solvent (pentane, diethyl ether, toluene, hexane and benzene). Prof. Wang's group also published the synthesis of metallo-organic particles using a very similar methodology [X. Sun, S. Dong, E. Wang, J. Am. Chem. Soc. 2005, 127, 13102]. New metallo-organic particles were synthesised by mixing H₂PtCl₆ (metal ion source) and p-phenylenediamine (organic ligand) in water. Also, a new synthetic pathway was published for obtaining metallo-organic particles formed on the basis of infinite coordination polymerisation of metal ions bound by organic ligands [E. Coronado, J. R. Galán-Mascarós, M. Monrabal-Capilla, J. García-Martínez, P. Pardo-Ibañez, Adv.25 Mater. 2007, 19, 1359; W. J. Rieter, K. M. L. Taylor, H. An, W. Lin, W. Lin,J. Am. Chem. Soc. 2006, 128, 9024]. These particles formed by ions of Fe and triazole ligands or ions of Gd and a dicarboxylic ligand were synthesised using the reverse micelle technique or based on microemulsions.

However, to date the use of these metallo-organic particles as encapsulators or transporters of other substances has not been described.

### DESCRIPTION OF THE INVENTION

### Brief Description

One aspect of the invention relates to a metallo-organic system, hereinafter metallo-organic system of the invention, comprising:
a) a metal ion salt or complex, belonging to the following group, transition series metal ions, preferably, zinc, copper, iron, cadmium, manganese, nickel and cobalt, and of the rare earths family, preferably, gadolinium, europium, terbium and uranium, aluminium and gallium.
b) at least, one organic ligand,
c) and a substance of interest to be encapsulated, belonging to the following group: a biological entity, a drug, a vaccine, a diagnostic contrast agent, a marker, an organic compound, an inorganic compound, a metallo-organic compound or a nanomaterial (nanoparticles, nanotubes, nanowires, nanocrystals) or nanodevices,
the latter being encompassed by the first two ones.

A preferred embodiment of the invention comprises a metallo-organic system wherein the metal ion complex is Zn(NO₃)₂·6H₂O.

Another preferred embodiment of the invention comprises a metallo-organic system wherein the organic ligand is 1,4-bis(imidazole-1-ylmethyl)benzene (Bix).

Another more preferred embodiment of the invention comprises a metallo-organic system wherein the organic ligand is 1,4-bis(imidazol-1-ylmethyl)benzene (Bix) and the metal ion complex is Zn(NO₃)₂·6H₂O (Examples 1, 2, 3 and 4).

Another preferred embodiment of the invention comprises the metallo-organic system of the invention functionalised by means of a species, for example an antibody, a bacteria, a virus, a cell, a protein, a sugar, DNA, a medicine, a drug, an organic compound, a fluorescent compound, an inorganic compound, a metallo-organic compound or a nanomaterial (nanoparticles, nanotubes, nanowires and nanocrystals).

Another aspect of the invention relates to a process for obtaining the metallo-organic system of the invention, hereinafter process of the invention, which comprises the following stages:
a) one stage of adding the different elements - metal ion salt or complex, organic ligand, and substance of interest - in a single reaction solution, which is under stirring, and which can be carried out in one of the following ways:
   i) by adding a metal ion salt or complex to a solution containing one or several organic ligands and the substance of interest to be encapsulated or vice-versa - upon adding the metal ion salt or complex to the solution containing one or several organic ligands the formation of the metallo-organic particle begins which automatically encapsulates the substance of interest that is present in the reaction medium (see Example 1);
   ii) by adding one or several organic ligands to the solution containing a metal ion salt or complex and the substance of interest to be encapsulated or vice-versa (see Example 3).
   iii) by adding the substance of interest to a solution containing a metal ion salt or complex and one or several organic ligands or vice-versa; or
   iv) by adding a metal ion salt or complex, organic ligand and substance of interest to a solvent wherein the metallo-organic system is insoluble or vice-versa;
b) stirring, for example through ultrasound of the solution of mixture obtained in a), preferably at room temperature, and
c) separation of the metallo-organic systems obtained in b) through centrifugation and subsequent redispersion.

Finally, another aspect of the invention relates to the use of the metallo-organic system, whether or not functionalised, of the present invention, for the release and/or protection and/or storage and/or variation of properties, for example, increasing the solubility or reducing the toxicity, of substances of interest.

A further aspect of the invention relates to the use of the metallo-organic system, whether or not functionalised, in the preparation of a diagnostic or therapeutic pharmaceutical composition or drug. And consequently, a pharmaceutical composition comprising the metallo-organic system of the invention also forms part of the invention.

A further aspect of the invention relates to the use of the metallo-organic system, whether or not functionalised, in the production of catalysts, sensors, contrast agents, biomarkers, magnetic semiconductors and magnetic recording devices.

### Detailed Description

The proposed invention centres on the description of a new metallo-organic system for the encapsulation and release of active substances as well as a process for the encapsulation of substances of a diverse nature (e.g. drugs, magnetic particles, drugs, proteins, etc.) in metallo-organic micro- and nano-systems.

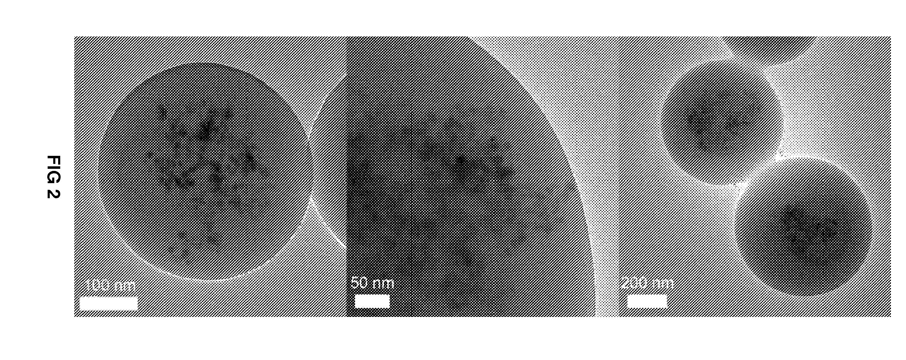

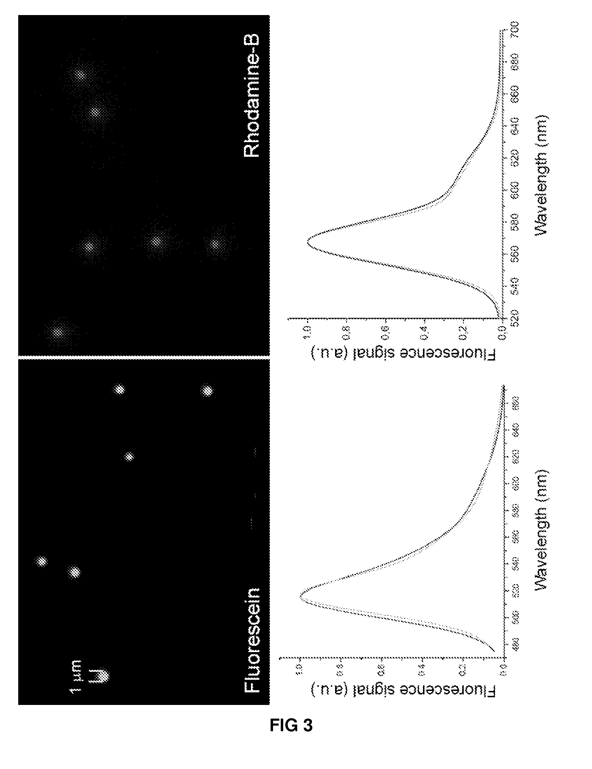

The present invention is based on the fact that the inventors have observed that it is possible, surprisingly, to encapsulate substances of interest by forming a metallo-organic system when a metal ion salt or complex (substance A), one or several organic ligands (referred to as B) and said substance of interest (C, understood as meaning one or more substances to be encapsulated) are mixed in a medium, through the immediate formation of a metallo-organic particle made up of A and B which encapsulates C formed through infinite coordination polymerisation, in other words, the binding of metal ions through the organic ligands (as in the case of organic polymers, this binding is infinite) - of metal ions joined by organic ligands. The metallo-organic systems thus obtained present a broad range of sizes, from 1 nm up to 100 micrometres, having obtained with the described examples a minimum size of 130 nm and preferably of between 150 nm and 5 microns. At the same time, as observed in Figure 2, the substance of interest remains encapsulated and protected inside the obtained metallo-organic system, thereby obtaining very well-defined spherical shapes (Figure 2 and 3) which enables easy identification and monitoring thereof.

It must be noted that to date, processes of conjugating metal ions with organic ligands were used to manufacture macroscopic metallo-organic compounds with applications in absorption, catalysis, ion exchangers, sensors, magnetism and optics, but nobody had carried out and verified that micro- and nano-capsules could also be formed for the purpose of encapsulating and releasing compounds of interest.

Also, it has been verified that one of the requirements for said methodology to function is that the metallo-organic particle made up of the metal ion salt or complex and one or several organic ligands has to be insoluble in the reaction medium because the microparticle is generated through rapid precipitation, a fairly common condition in the formation of metallo-organic particles [Chemically tailorable nanoparticles realized through metal-metalloligand coordination chemistry. C. A. Mirkin, M. Oh, B.-K. Oh, WO2007053181]. If the metal ion salt or complex and the organic ligand are soluble in the final reaction medium, the micro- and nano-particles are not formed.

The metallo-organic system of this invention for the encapsulation and/or release of substances not only adds a new system to already existing ones - liposomes, spherical particles of organic polymers, phospholipid micelles, cyclodextrins, carbon nano-particles and dendrimers - but also allows new functional systems to be obtained when combining the properties inherent to the metallo-organic particles (such as porosity, magnetism, electronic, fluorescence, etc.) with the benefits and applications offered by encapsulation and the properties of the encapsulated substances, whether medicines, magnetic particles, drugs, proteins, diagnostic contrast agents, vaccines, etc. For example, with said invention, systems can be obtained for use in the release of drugs (see Example 4), storage and/or protection of diverse substances, such as sensors, biomarkers, detection agents (see Example 2), magnetic semiconductors and magnetic recording and, consequently, with applications in sectors as diverse as medicine, electronics, catalysis, the environment, etc.

Therefore, one aspect of the invention relates to a metallo-organic system, hereinafter metallo-organic system of the invention, which comprises:
a) a metal ion salt or complex, belonging to the following group: transition series metal ions, preferably, zinc, copper, iron, cadmium, manganese, nickel and cobalt, and of the rare earths family, preferably, gadolinium, europium, terbium and uranium, aluminium and gallium.
b) at least, one organic ligand, and
c) a substance of interest to be encapsulated, belonging to the following group: a biological entity, a drug, a vaccine a diagnostic contrast agent, a marker, an organic compound, an inorganic compound, a metallo-organic compound or nanomaterial (nanoparticles, nanotubes, nanowires, nanocrystals) or nanodevices, the latter being encompassed by the first two.

As used in the present invention, the term "metal ion salt or complex" relates to transition series metal ions, such as zinc (see Examples 1, 2, 3 and 4), copper, iron, cadmium, manganese, nickel and cobalt, of the rare earths family, for example, gadolinium, europium, terbium and uranium, aluminium and gallium.

As used in the present invention, the term "organic ligand" relates to an organic compound with one or more functional groups, wherein the functional group(s) may be carboxylic acids, phosphoric groups, alcohols, thiols amines, catechols and any functional group derived from nitrogen, such as Bix for example (Chemically tailorable nanoparticles realized through metal-metalloligand coordination chemistry. C. A. Mirkin, M. Oh, B.-K. Oh, W02007053181).

A preferred embodiment of the invention comprises a metallo-organic system wherein the metal ion complex is Zn(NO₃)₂·6H₂O.

Another preferred embodiment of the invention comprises a metallo-organic system wherein the organic ligand is 1,4-bis(imidazol-1-ylmethyl)benzene (Bix).

A more preferred embodiment of the invention comprises a metallo-organic system wherein the organic ligand is 1,4-bis(imidazol-1-ylmethyl)benzene (Bix) and the metal ion complex is Zn(NO₃)₂·6H₂O (see Examples 1, 2, 3 and 4).

Another preferred embodiment of the invention comprises a metallo-organic system wherein the biological entity to be encapsulated belongs to the following group: bacteria, virus, eukaryote cell, protein, antibody, sugars, DNA and RNA.

The nano- and microcapsules or metallo-organic systems described in the present invention may come in different sizes, with minimum diameters of 130 nm having been reached.

On a separate note, for applications wherein the recognition of specific biological targets is necessary, the metallo-organic system of the invention may be functionalised on its outer surface using another species (substance), such as an antibody, to direct said system towards the target tissues, for example, tumoral cells. In this sense, the functionalisation of the metallo-organic system of the invention can be carried out by adding a solution of the species that serves to functionalise (for example, an antibody, a fluorescent compound, etc.) within the dispersion that contains the already synthesised metallo-organic system. By means of interactions that may be of the van der Waals type, electrostatic, hydrogen bridge, π-π-, coordination or covalent, said species interact with the metallo-organic systems functionalising their surface.

Another preferred embodiment of the invention relates to a metallo-organic system of the invention functionalised by means of a species, for example an antibody, a bacteria, a virus, a cell, a protein, a sugar, DNA, a medicine, a drug, an organic compound, a fluorescent compound, an inorganic compound, a metallo-organic compound or a nanomaterial (nanoparticles, nanotubes, nanowires and nanocrystals).

Another aspect of the invention relates to a process for obtaining the metallo-organic system of the invention, hereinafter process of the invention, which comprises the following stages:
a) one stage of adding the different elements - metal ion salt or complex, organic ligand, and substance of interest - in a single reaction solution, which is under stirring, and which can be carried out in one of the following ways:
   i) by adding a metal ion salt or complex to a solution containing one or several organic ligands and the substance of interest to be encapsulated or vice-versa - upon adding the metal ion salt or complex to the solution containing one or several organic ligands the formation of the metallo-organic particle begins which automatically encapsulates the substance of interest that is present in the reaction medium (see Example 1);
   ii) by adding one or several organic ligands to the solution containing the metal ion salt or complex and the substance of interest to be encapsulated or vice-versa (see Example 3).
   iii) by adding the substance of interest to a solution containing the metal ion salt or complex and one or several organic ligands or vice-versa; or
   iv) by adding the metal ion salt or complex, organic ligand and substance of interest to a solvent wherein the metallo-organic system is insoluble or vice-versa;
b) stirring, which can be carried out by any method known by stirring, for example through ultrasound or mechanical stirring of the solution of mixture obtained in a), preferably at room temperature, and
c) separation of the metallo-organic systems obtained in b) through any method known by an expert in the art, for example centrifugation, separation of the phases, filtration, attraction by means of external stimuli or precipitation and their subsequent redispersion

The size of these metallo-organic systems can be controlled by varying the reaction conditions, for example, by modulating the concentrations of the initial solutions (of the metal and organic ligand) (see Example 6). At the same time, the dimensions could also be modulated by controlling the speed of adding said solutions. In general, the guidelines are:
- the higher the speed of addition, the smaller the metallo-organic systems (see non-encapsulated metallo-organic particles published in Angewandte Chemie Internacional Edition 2008, 47, 1857)
- the higher the concentration generally entails obtaining smaller metallo-organic systems, and the lower the concentration, the greater (see Example 6, Figure 8).

In this way, depending on the specific metallo-organic system of the invention and required size, an expert in the art with the information provided in the present invention could easily design the appropriate reaction conditions.

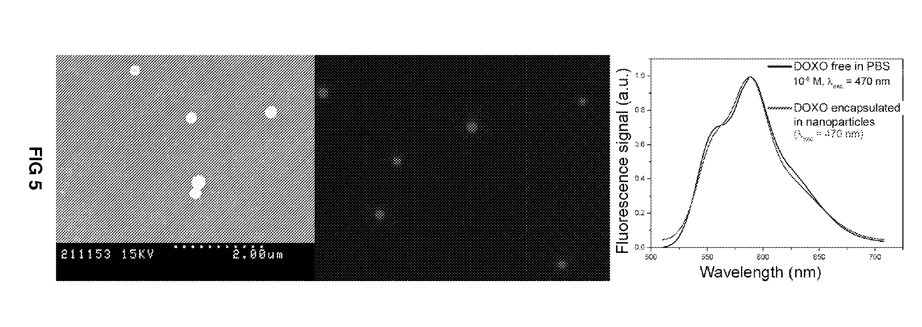

Among the sectors to use these metallo-organic systems, medicine is the field in which these micro- and nano-capsules have the most potential given their capacity to encapsulate medicines, vaccines, drugs, peptides, proteins or DNA molecules so as to then release them in a controlled and precise manner in the tissues or specific points of the human body. Within this field, the invention presented herein allows new systems to be obtained with advantages in respect of the traditional systems. By way of an example, with the use of metal ions of the rare earths family, metallo-organic particles are obtained that emit fluorescence at wavelengths of more than 500 nm (the limit above which in general cells, organs and tissues do not emit fluorescence), and in which the required substances are encapsulated (see Example 4 and Figure 5). In this case, the intrinsic fluorescence of the metallo-organic particles enables their monitoring inside live organisms during the transport process within the human body and during the release of the substance or in cells *in vitro.* Another practical example within said sector would be drugs encapsulated in metallo-organic particles which act simultaneously as contrast agents and as advanced drug release systems. These particles, synthesised with ions such as Gd(III) or Eu(III), allow simultaneous detection, monitoring and treatment of diseases. Also, if said particles are functionalised or sectorised with an appropriate antibody, the final system can recognise specific sites of the body and thereby only act on specific places.

Finally, another aspect of the invention relates to the use of the metallo-organic system of the present invention, whether or not functionalised, for the release and/or protection and/or storage and or variation of properties, for example, to increase the solubility or reduce the toxicity of substances of interest.

A further aspect of the invention relates to the use of the metallo-organic, whether or not functionalised, in the production of a diagnostic or therapeutic pharmaceutical composition or medicament. And consequently, a pharmaceutical composition comprising the metallo-organic system of the invention also forms part of the present invention.

Another aspect of the invention relates to the use of the metallo-organic system, whether or not functionalised, in the production of catalysts, sensors, contrast agents, biomarkers, magnetic semiconductors and devices for magnetic recording.

Throughout this description and the claims, the word "comprises" and variants thereof do not intend to exclude other technical characteristics, additives, components or steps. For persons skilled in the art, other objects, benefits and characteristics of the invention will be inferred partly from the description and partly from the practice of the invention. The following examples and drawings are provided by way of illustration, and are not intended to limit the present invention.

### DESCRIPTION OF THE DRAWINGS

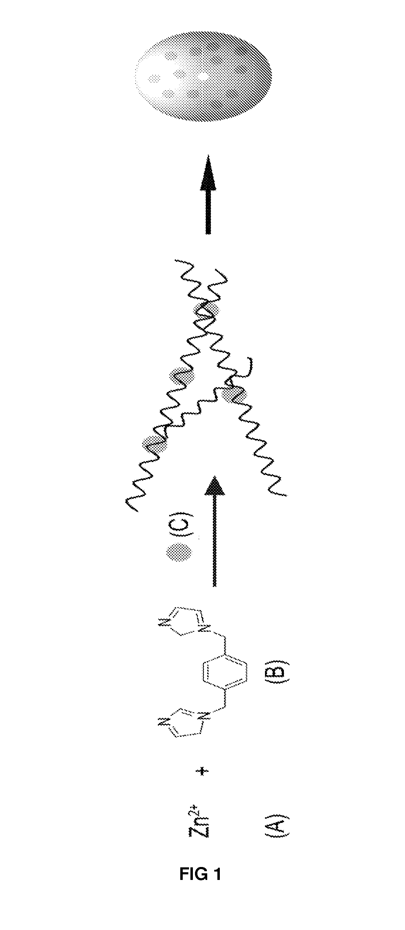

**Fig. 1** is a descriptive drawing of the formation process of the metallo-organic system of the invention that encapsulates substances. Encapsulation occurs when the species to be encapsulated (C) is present in the medium wherein the metallo-organic systems are manufactured synthesised by means of a process that mixes a metal ion (A) and an organic ligand (C) **Fig. 2** shows the images obtained by transmission electron microscopy of the magnetic nanoparticles of Fe₃O₄ (20 nm mean diameter; Fluka) encapsulated within the metallo-organic system. The images have been obtained using a JEOL TEM 2010F transmission electron microscope. White bars are included as a size reference.
**Fig. 3** shows fluorescent microscopy images of the metallo-organic system of the invention with encapsulated fluorescent organic molecules (fluoroscein (Fluka) in green and Rhodamine-B (Sigma-Aldrich) in red). Both images have been obtained using an AxioObserver Z1M (Zeiss Microscope) optical microscope. The bottom part shows the corresponding fluorescence emission spectrum (green line) which presents a very similar shape to that of the species encapsulated in free state (red line). These spectrums have been obtained using an Applied Photophysics fluorometer system with a Quantel pulsed laser, and demonstrate that the encapsulated substances are not modified during the encapsulation process.

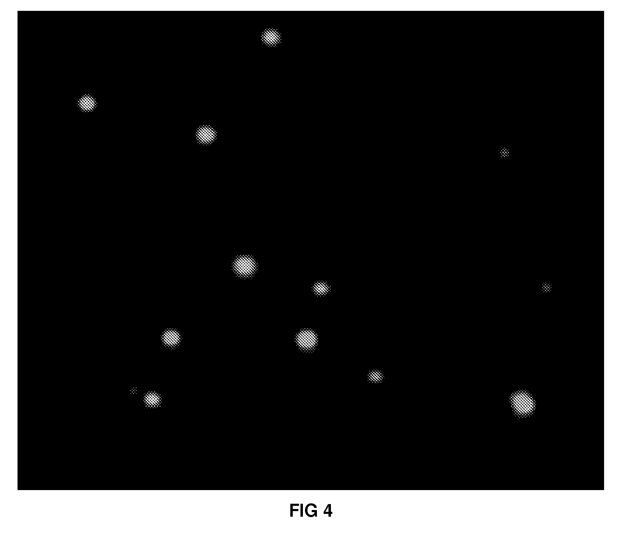

**Fig. 4** shows a fluorescent microscopy image of the metallo-organic system of the invention with encapsulated fluoroscein (Fluka) synthesised according to version ii of the method of the invention. Said image has been obtained using an AxioObserver Z1 M (Zeiss Microscope) optical microscope.
**Fig. 5** shows, on the left hand side, scanning microscopy images (left hand corner of the image) and of fluorescence of the metallo-organic system wherein the antitumoral drug doxorubicin has been encapsulated. The right hand side shows the corresponding fluorescence emission spectrum from which it can be observed that the doxorubicin has not been modified during the encapsulation process (black line - DOXO in PBS - versus red line - encapsulated DOXO). The scanning and fluorescence images have been taken using a Hitachi S-570 scanning microscope and an AxioObserver Z1M (Zeiss 25 Microscope) optical microscope, respectively. The fluorescence intensities have been obtained using an Applied Photophysics fluorometer with a Quantel pulsed laser.

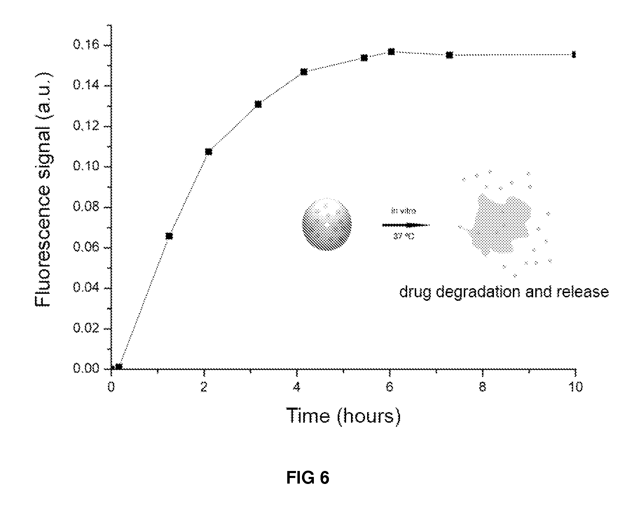

**Fig. 6** shows the monitoring through fluorescence of the release of doxorubicin from the metallo-organic system wherein the doxorubicin was previously encapsulated. The figure contains an illustrative sketch of the drug release process in said metallo-organic system. The fluorescence emission spectrum has been acquired using an Applied Photophysics fluorometer with a Quantel pulsed laser.

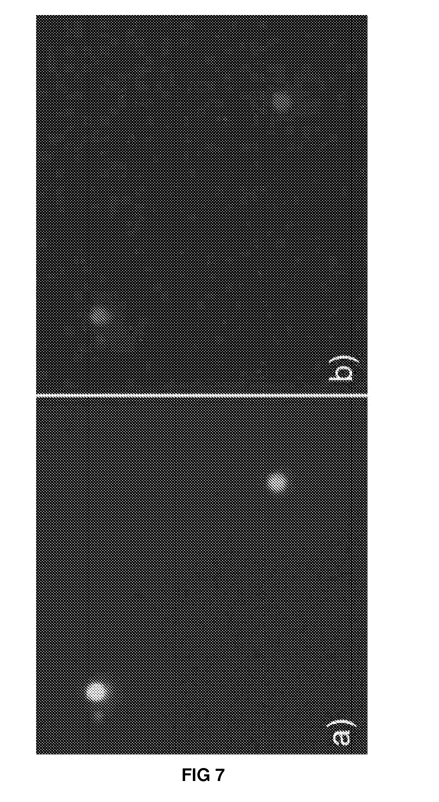

**Fig. 7** shows fluorescence microscopy images of the metallo-organic system of the invention with encapsulated fluoroscein (Fluka) in green (left hand side of the image) and functionalised Rhodamine-B (Sigma-Aldrich) in red (right hand side of the image). Both images have been obtained using an AxioObserver Z1M (Zeiss Microscope) optical microscope.
**Fig. 8** shows an image of the measurements taken using the light scattering technique of the different metallo-organic systems manufactured varying the concentrations of the solutions of ions Zn(II) in the organic ligand Bix. Said measurements have been taken using a Malvern Zetasizer Nano-ZS differential light scattering equipment.

### EXAMPLES OF EMBODIMENT OF THE INVENTION

The following examples are provided to illustrate and not to limit the claims of the invention.

### Example 1.- Encapsulation of magnetic nanoparticles of Fe₃O₄ in the metallo-organic system of the invention

The methodology presented herein allows magnetic nanoparticles of Fe₃O₄ to be encapsulated in metallo-organic systems. According to the procedure employed (version i of the process of the invention), the nanoparticles of Fe₃O₄ encapsulated in metallo-organic systems are obtained through addition, at room temperature and under stirring in ultrasound (during 10 minutes), of a solution (water; 5 ml; purity milli-Q) of Zn(NO₃)₂·6H₂O (150 mg; 98 % purity, Sigma-Aldrich) to a solution (absolute ethanol; 25 ml; ROMIL) that contains the organic ligand 1,4-bis(imidazol-1-ylmethyl)benzene (Bix; 121 mg; synthesised following the procedure described by Dhal, (P. K. Dhal, F. H. Arnold, Macromolecules, 1992, 25, 7051) and the scattered magnetic nanoparticles of Fe₃O₄ (0.1 ml; 20 nm mean diameter; Fluka) (Fig 1). Immediately, precipitation is observed of a scattered brown solid, of the colour of the Fe₃O₄ nanoparticles, which are already encapsulated in the metallo-organic systems of the invention.

The metallo-organic systems with the encapsulated nanoparticles are separated by centrifugation (1000 RPM; 15 minutes) and re-scattered with ethanol (absolute ethanol; 15 ml; ROMIL). This process is repeated several times, more specifically up to four times (the washing cycles are important for obtaining pure metallo-organic systems with the encapsulated substance, although the number of washes is random, for some systems one cycle would be sufficient whereas others would require several cycles, which in any case is easy to determine for an expert).

Finally, the separated and washed systems can be preserved in a solid state or re-scattered in ethanol or in a saline phosphate buffer solution in order to finally obtain a colloidal solution of said encapsulated particles (Fig 2).

The images obtained through transmission electron microscopy demonstrate that the magnetic nanoparticles of Fe₃O₄ (substance of interest) are encapsulated inside the metallo-organic system of the invention forming almost perfectly spherical shapes of between approximately 300-500 nm.

### Example 2.- Encapsulation of fluorescent organic molecules in the metallo-organic system of the invention.

According to the method used (version i of the process of the invention), the fluorescent organic molecules encapsulated in metallo-organic systems are obtained through adding, at room temperature and under stirring (10 minutes), a solution containing the metal ion (water; 5 ml; purity milli-Q) of Zn(NO₃)₂·6H₂O (150 mg; 98% purity, Sigma- Aldrich) to a solution (absolute ethanol; 25 ml; ROMIL) that contains the organic ligand 1,4-bis(imidazol-1-ylmethyl)benzene (Bix; 121 mg; synthesised following the procedure described by Dhal, (P. K. Dhal, F. H. Arnold, Macromolecules, 1992, 25, 7051) and a dissolved fluorescent organic molecule such as fluoroscein (0.4 mg; Fluka) or Rhodamine-B (0.5 mg; Sigma-Aldrich). Immediately, precipitation is observed of a scattered solid of the colour of the encapsulated fluorescent molecule.

The metallo-organic systems with the encapsulated fluorescent molecules are separated by centrifugation (1000 RPM; 15 minutes) and re-scattered with ethanol (absolute ethanol; 15 ml; ROMIL). This process is repeated four times. Finally, the separated and washed systems can be preserved in solid state or re-scattered in ethanol or in a saline phosphate buffer solution so as to finally obtain a colloidal solution of said encapsulated particles (Fig 3).

As can be observed, metallo-organic systems are obtained with very well-defined spherical shapes ranging in size between 0.5-1 microns with a fairly homogeneous size. At the same time, spectrum reading demonstrates that the encapsulated substances are not modified or degraded during the encapsulation process meaning that they can be identified and monitored during their subsequent use (Fig 3). The metallo-organic systems obtained in this example were stored during 5 months as a colloidal solution in ethanol, and remained stable after this time, as well the fluoroscein.

### Example 3. - Encapsulation of fluoroscein in the metallo-organic system of the invention.

According to the method used (version ii of the process of the invention), the fluoroscein encapsulated in metallo-organic systems is obtained by adding, at room temperature and under stirring (10 minutes), a solution (absolute ethanol; 25 ml; ROMIL) containing the organic ligand 1,4-bis(imidazol-1-ylmethyl)benzene (Bix; 121 mg; synthesised following the procedure described by Dhal, (P. K. Dhal, F. H. Arnold, Macromolecules, 1992, 25, 7051) to a solution (water (5ml; purity milli-Q)/absolute ethanol (5 ml; ROMIL) containing the metal ion Zn(NO₃)₂·6H₂O (150 15 mg; 98% purity, Sigma-Aldrich) and dissolved fluoroscein (0.4 mg; Fluka) (Fig 4). Immediately, precipitation is observed of a scattered solid of the colour of the encapsulated fluorescent molecule.

The metallo-organic systems with the encapsulated fluoroscein are separated by centrifugation (1000 RPM; 15 minutes) and re-scattered with ethanol (absolute ethanol; 15 ml; ROMIL). This process is repeated four times. Finally, the separated and washed systems can be preserved in solid state or re-scattered in ethanol or in saline phosphate buffer solution in order to finally obtain a colloidal solution of said encapsulated particles (Fig 4).

As can be observed, metallo-organic systems are obtained with very well defined spherical shapes of a size between 0.5-1 microns with a fairly homogeneous size.

### Example 4.- Encapsulation of doxorubicin in the metallo-organic system of the invention and its controlled release.

According to the method used (version i of the process of the invention), the organic molecule doxorubicin (antitumoral drug) encapsulated in metallo-organic systems is obtained by adding, at room temperature and under stirring (10 minutes), a solution containing the metal ion (water; 5 ml; purity milli-Q) of Zn(NO₃)₂·6H₂O (150 mg; 98 % purity, Sigma-Aldrich) to a solution (absolute ethanol; 25 ml; ROMIL) containing the ligand 1,4-bis(imidazol-1-ylmethyl)benzene (Bix; 121 mg; synthesised following the procedure described by Dhal, (P. K. Dhal, F. H. Arnold, Macromolecules, 1992, 25, 7051) and the doxorubicin (0.5 mg; Fluka) (Fig 1). Immediately, precipitation is observed of a scattered solid of the colour of doxorubicin.

The metallo-organic systems with the encapsulated doxorubicin are separated by centrifugation (1000 RPM; 15 minutes) and re-scattered with ethanol (absolute ethanol; 15 ml; ROMIL). This process is repeated four times. Finally, the separated and washed systems can be preserved in solid state, or re-scattered in ethanol or in a saline phosphate buffer in order to finally obtain a colloidal solution of said encapsulated particles (Fig 5).

As can be observed, metallo-organic systems are obtained with very well defined spherical shapes of a size between 200-300 nm and with a fairly homogenous size. At the same time, reading of the spectrums demonstrates that the encapsulated doxorubicin has not been modified or degraded during the encapsulation process meaning that it can continue to provide its biological activity during its subsequent use (Fig 5).

At the same time, and in therapeutic applications for example, the drug's release by the metallo-organic system of the invention is important. Thus, the process of controlled release *in vitro* was studied by placing a sealed dialysis bag (Mw= 3000; Spectrum Lab) containing 3 ml of concentrated dispersion in saline phosphate buffer solution (Sigma-Aldrich) of the previously obtained metallo-organic system with encapsulated doxorubicin. This bag was dialysed with 100 ml of saline phosphate buffer solution (Sigma Aldrich) under stirring (continued throughout the process). The amount of released doxorubicin was controlled every hour by collecting 2 ml of the PBS solution external to the bag and measuring the evolution of the fluorescent signal. This evolution reveals a continued release of the drug during 8 hours (Fig 6).

### Example 5.- Functionalisation with Rhodamine B of a metallo-organic system with previously encapsulated fluoroscein.

Starting out with a colloidal solution of the metallo-organic systems wherein fluoroscein had been previously encapsulated (synthesised according to Example 2), functionalisation of the external surface of said systems is carried out by adding, at room temperature and under stirring (10 minutes), a solution (absolute ethanol; 1 ml; ROMIL) containing Rhodamine (C ∼ 10₋₃ M; Sigma-Aldrich) to the colloidal solution (absolute ethanol; 5 ml; ROMIL) containing the metallo-organic systems with encapsulated fluoroscein. The metallo-organic systems with encapsulated fluoroscein and functionalised with Rhodamine B are separated by centrifugation (1000 RPM; 10 minutes) and re-scattered using ethanol (absolute ethanol; 5 ml; ROMIL). This process is repeated four times. Finally, the separated and washed systems can be preserved in solid state or re-scattered in ethanol (Fig 7).

As can be observed, metallo-organic systems are obtained with a spherical shape, which have retained their size of 0.5 - 1 µm. A study of the fluorescence images (Figure 7) indicates that both fluorescent molecules (the encapsulated fluoroscein and Rhodamine B on the surface) form part of the final functionalised metallo-organic system.

### Example 6.- Control of the size of the metallo-organic systems of the invention with encapsulated fluoroscein.

According to the method used (version i of the process of the invention), the dimensions of the metallo-organic systems of the invention wherein fluoroscein has been encapsulated is modulated by varying the concentrations of the reaction components. In a general procedure, the encapsulation of fluoroscein in the metallo-organic systems is carried out by adding, at room temperature and under stirring (10 minutes), a solution containing the metal ion (water; purity milli-Q) of Zn(NO₃)₂·6H₂O (98% purity, Sigma-Aldrich) to a solution (absolute ethanol; ROMIL) that contains the organic ligand 1,4-bis(imidazol-1-ylmethyl)benzene (Bix; synthesised following the procedure described by Dhal, (P. K. Dhal, F. H. Arnold, Macromolecules, 1992, 25, 7051) and fluoroscein (0.4 mg; Fluka). Immediately, precipitation is observed of a scattered solid of the colour of the encapsulated fluorescent molecule, and absolute ethanol is added (100 ml; ROMIL) in order to stabilise the formed metallo-organic systems. Thus, depending on the concentration of the metal ion and the organic ligand Bix used in said general procedure, the size of the metallo-organic systems will vary according to Table 1.

**Table 1.**

| Amount of Zn(NO₃)·6H₂O (mg) | Volume of H₂O (ml) | Amount of Bix (mg) | Volume of Ethanol (ml) | Amount of Fluoroscein (mg) | Size of the Metallo-organic system (nm) |
|---|---|---|---|---|---|
| 150 | 20 | 120 | 100 | 0.4 | 1050 |
| 150 | 10 | 120 | 50 | 0.4 | 600 |
| 150 | 5 | 120 | 20 | 0.4 | 270 |
| 150 | 1 | 120 | 5 | 0.4 | 130 |

As can be observed, metallo-organic systems are obtained with very well defined spherical shapes whose sizes have been calculated using transmission electron microscopy and laser scattering measurements (Fig 8).

## Claims

1. Metallo-organic system **characterised in that** it comprises:
a) a metal ion salt or complex selected from among transition series metals of the family of rare earths, aluminium or gallium,
b) at least, one organic ligand, and
c) a substance selected from the group comprising: a biological entity, a drug, a vaccine, a diagnostic contrast agent, a marker, an organic compound, an inorganic compound, a metallo-organic compound or a nanomaterial,
substance (c) being encapsulated by the first two (a) and (b).

2. Metallo-organic system according to claim 1, wherein the metal ion salt or complex is selected from the list comprising zinc, copper, iron, cadmium, manganese, nickel, cobalt, gadolinium, europium, terbium, uranium, aluminium or gallium.

3. Metallo-organic system according to either claim 1 or 2, wherein the metal ion complex is Zn(NO₃)₂·6H₂O.

4. Metallo-organic system according to any of claims 1 to 3, wherein the organic ligand is an organic compound with one or more functional groups, wherein the functional group(s) are selected from the list comprising carboxylic acids, phosphoric groups, alcohols, thiols, amines, catechols and any functional group derived from nitrogen.

5. Metallo-organic system according to claim 4, wherein the organic ligand is 1,4-bis(imidazol-1-ylmethyl)benzene (Bix).

6. Metallo-organic system according to any of claims 1 to 5, **characterised in that** the encapsulated substance is a biological entity selected from the list comprising a bacteria, a virus, a eukaryote cell, a protein, an antibody, sugars, DNA or RNA.

7. Metallo-organic system according to any of claims 1 to 5, wherein the encapsulated substance is a nanomaterial selected from the list comprising nanoparticles, nanotubes, nanowires, nanocrystals or nanodevices.

8. Metallo-organic system according to any of claims 1 to 7, **characterised in that** it is functionalised on its outer surface with another species or substance.

9. Metallo-organic system according to claim 8, wherein the species or substance is selected from the group comprising an antibody, a bacteria, a virus, a cell, a protein, a sugar, DNA, a medicine, a drug, an organic compound, a fluorescent compound, an inorganic compound, a metallo-organic compound or a nanomaterial.

10. Process for obtaining the metallo-organic system according to any of claims 1 to 9, **characterised in that** it comprises the following stages:
a) adding the different elements - metal ion salt or complex, organic ligand and substance to be encapsulated - to a reaction solution, which is under stirring,
b) stirring of the solution of mixture obtained in (a), and
c) separation of the metallo-organic systems obtained in (b),
wherein the metallo-organic system resulting from the mixture of the metal ion salt or complex and the organic ligand added in step (a) are insoluble in the reaction solution.

11. Process according to claim 10, wherein step (a) is carried out by adding a metal ion salt or complex to a solution containing one or several organic ligands and the substance to be encapsulated or vice-versa.

12. Process according to claim 10, wherein step (a) is carried out by adding one or several organic ligands to the solution containing the metal ion salt or complex and the substance to be encapsulated or vice-versa.

13. Process according to claim 10, wherein the substance to be encapsulated is added in step (a) to a solution containing the metal ion salt or complex and one or several organic ligands or vice-versa.

14. Process according to claim 10, wherein the addition of step (a) is carried out by adding the metal ion salt or complex, organic ligand and substance of interest to a solvent wherein the metallo-organic system is insoluble or vice-versa.

15. Process according to any of claims 10 to 14, wherein the stirring of step (b) is carried out at room temperature.

16. Process according to any of claims 10 to 15, wherein the separation of step (c) is carried out through centrifugation and subsequent re-scattering.

17. Use of the metallo-organic system according to any of claims 1 to 9, for the release and/or protection and/or storage and/or variation of the properties of the encapsulated substances.

18. Use of the metallo-organic system according to any of claims 1 to 9, to produce a diagnostic or therapeutic pharmaceutical composition or medicament.

19. Diagnostic or therapeutic pharmaceutical composition **characterised in that** it comprises the metallo-organic system according to claims 1 to 9.

20. Use of the metallo-organic system according to any of claims 1 to 9, in the production of catalysts, sensors, contrast agents, biomarkers, magnetic semiconductors or devices for magnetic recording.
